Europäisches Patentamt

European Patent Office

Office européen des brevets

(19)

(11) Publication number: **0 271 935**
**A2**

# EUROPEAN PATENT APPLICATION

(21) Application number: 87202153.0

(51) Int. Cl.4 **G01N 33/573**

(22) Date of filing: 05.11.87

(30) Priority: **17.11.86 NL 8602915**

(43) Date of publication of application:
**22.06.88 Bulletin 88/25**

(84) Designated Contracting States:
**AT BE CH DE ES FR GB GR IT LI LU NL SE**

(71) Applicant: **Innogenetics N.V.**
**Industriepark Zwijnaarde 7 Box 4**
**B-9710 Gent(BE)**

(72) Inventor: **de Broe, Marc Eduard**
**Hillare 10**
**B-9100 Lokeren(BE)**

(74) Representative: **Prins, Hendrik Willem et al**
**Octrooibureau Arnold & Siedsma**
**Sweelinckplein, 1**
**NL-2517 GK The Hague(NL)**

(54) **Method and diagnostic kit for diagnosis and/or prospective following of injury at the point of the pulmonary microcirculation.**

(57) The invention relates to the use of a specific antibody for an antigen which is secreted by the alveolar lumina in a biological material under the influence of injury at the point of the pulmonary microcirculation, for the manufacture of a diagnostic kit for diagnosis and/or prospective following of this injury at the point of said pulmonary microcirculation and to a method for diagnosis and/or prospective following of injury at the point of the pulmonary microcirculation, comprising the immunological determination of said antigen or antigens secreted by the alveolar lumina microcirculation under influence of this injury.

Preferably the antigen is placental alkaline phosphatase or $\beta_2$ microglobulin.

FIG.2

EP 0 271 935 A2

# Method and diagnostic kit for diagnosis and/or prospective following of injury at the point of the pulmonary microcirculation.

The current invention relates to a method for diagnosis and/or prospective following of injury at the point of the pulmonary microcirculation, and to a diagnostic kit for use thereby.

Injury at the point of the pulmonary microcirculation is considered to be primarily the result of a cytointoxication which has been induced by cytotoxic oxides, primarily oxygen radicals like the superoxide radical, the hydroxyl radical, singlet oxygen, peroxide radicals of lipids and hydrogen peroxide (Lee Frank, Oxidant Injury to Pulmonary Endothelium, in Sami I. Said, The Pulmonary Circulation and Acute Lung Injury, 1985, pp. 283-305). Under normal conditions these toxic oxygen metabolites are present in quantities such that no harmful consequences arise. Under conditions of hyperoxidant stress the quantity of toxic oxygen metabolites grows to such an extent that an insufficient detoxification takes place. Unless the intracellular balance is restored, particularly morphological deviations ensue at the point of the microcirculation for the lung. There is a difference, however, in susceptibility of the different cell types of this microcirculation. In terms of decreasing sensitivity to oxygen-induced cytotoxicity the cell types can be classified as follows: endothelial cells > type I epithelial cells (membranous pneumocytes) ≫ type II epithelial cells (granular pneumocyte) and interstitial cells. In other words, pulmonary oxygen intoxication first manifests itself in damage/death of endothelial cells and type I epithelial cells, as a result of which capillary leakage and edema can ensue.

Several researchers have examined whether the meta bolic function of pulmonary endothelial cells can be used for detection of lung injury at an early stage. An effort was made in one study to find a correlation between endothelial injury and the enzyme localized on the luminal surface of the pulmonary endothelium and which converts angiotensin I to angiotensin II. In a recent study the possibility has been further examined as to whether measurements of the plasmaantigen which is related to factor VIII and occurs in plasma of patients with acute respiratory distress syndrome (ARDS) is suitable as an early marker of pulmonary injury.

No clear and diagnostically relevant conclusions can be drawn from any of these studies. Consequently there is an urgent need of sensitive markers of injury at the point of the pulmonary microcirculation.

The present inventors have discovered that placental alkaline phosphatase and $\beta_2$ microglobulin are useful as sensitive markers of injury at the point of the pulmonary microcirculation and are highly suitable for diagnostic and prospective purposes.

## 1. Placental alkaline phosphatase

Human alkaline phosphatase (E.C. 3.1.3.1.) (hPLAP) is a glycoprotein which occurs in a number of different isoenzymatic forms, which are made after the organs from which they are derived, such as bone, kidney, liver and placenta.

The current inventors have detected a correlation between the total quantity of cigarettes smoked daily (x) and the concentration of human placental alkaline phosphatase (hPLAP) in the serum (y), namely:

$y = (2.56x + 17.3)/1000.$

The hPLAP activity decreases rapidly when smoking is stopped, but increases again when smoking is resumed. In order to find the possible sources from which hPLAP is released with smoking, a large number of tissues were examined. The normal lung containing hPLAP activity, was found to be the organ with the highest eutopical expression of this enzyme. An increased concentration of hPLAP in lung tissue of smokers compared to non-smokers could not be detected, which indicates that smoking has no influence on the total concentration of hPLAP in the lung but influences the release of this enzyme.

The effects of smoking on the lung correspond to those in other clinical situations where injury at the point of the pulmonary microcirculation is present. In further investigations the researchers have been able to demonstrate an increased concentration of hPLAP in serum in the case of acute respiratory distress syndrome in adults and children, artificial ventilation, intoxication with a compound which causes pulmonary oxygen intoxication, namely paraquat, and extracorporeal circulation with bio-incompatible membranes.

## Acute respiratory distress syndrome (ARDS) in adults and children

The following criteria were applied in the diagnosis of ARDS (Ognibene F.P. et al, New England Journal of Medicine, 315-547, 1986):

- the occurrence of a precipitating event
- diffuse bilateral pulmonary infiltrate or a chest X-ray photo
- normal intravascular volume (reflected by a "wedge pressure" (Capillary pulmonary pressure) of less than 18 mmHg
-arterial hypoxemia

Twelve patients with ARDS diagnosed on the basis of the above cited criteria were examined. At the moment of diagnosis the serum hPLAP was in all cases greater than 0.1 U/L (cut-off value) and remained greater during the subsequent days. In a prospective examination of patients in the high risk group for ARDS who were followed daily, an increase of serum hPLAP was observed in those patients developing the disease.

Artificial ventilation

In eighteen patients an increase or an increased serum hPLAP value was observed during this treatment. The examined cases of artificial ventilation belonged to different types varying from conventional artificial ventilation to positive and expiratory pressure (PEEP) ventilation with a $FIO_2$ - (fractioned, inspiratory oxygen tension) of 0.21 - 1.0.

Paraquat poisoning

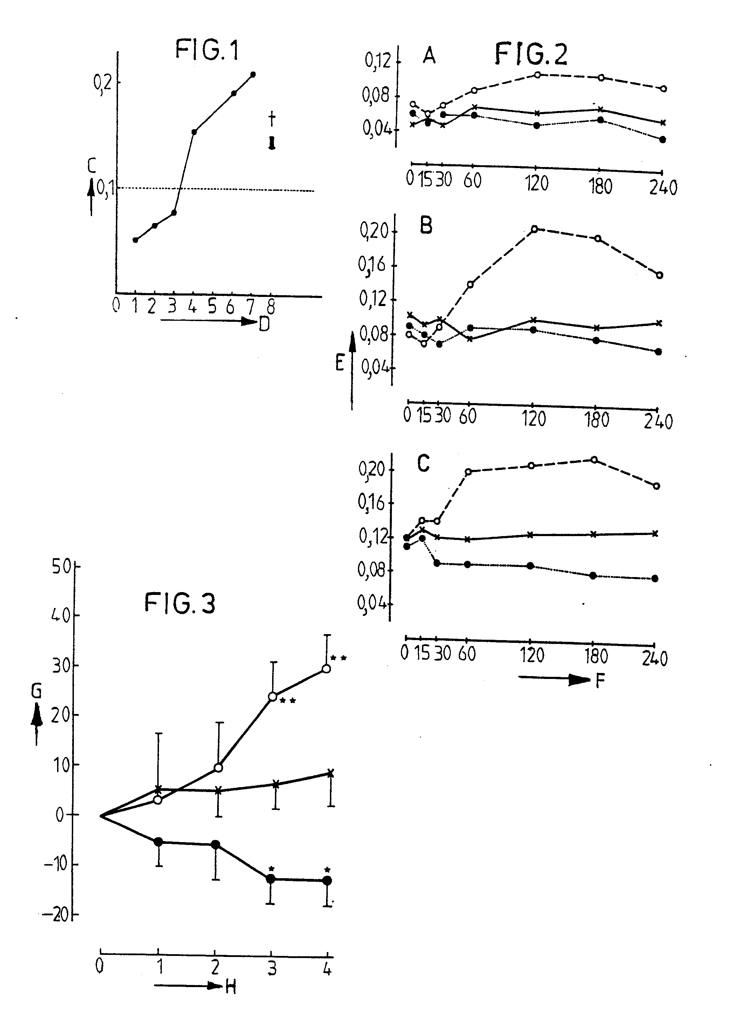

In two patients (born 1967 and 1960) who had attempted suicide using paraquat, the serum hPLAP was followed prospectively. Fig. 1 shows for the first patient the course of the serum hPLAP concentration in days after the ingestion of paraquat, whereby the serum paraquat concentration amounted to 85 $\mu$g/ml at the moment of ingestion. The serum hPLAP concentration rose above the normal value from day 4, on which day pulmonary complications appeared. The second patient had ingested only a small amount of paraquat and vomited shortly after admission and had a paraquat concentration of only 0.4 $\mu$g/ml. He survived the suicide attempt yet displayed a brief and ephemeral increase of hPLAP in serum.

Extracorporeal circulation with bio-incompatible membranes

Extracorporeal circulation can be performed using bio-compatible membranes (e.g. polyacrylonitrile and modified cellulose) or bio-incompatible membranes (e.g. cuprophane). Fig. 2A-C shows for three patients the course of the serum hPLAP concentration (E), as function of the type of membrane used (o----o cuprophane, x_____x modified cellulose, ●.....● polyacrylonitrile). In each of the cases an increase in serum hPLAP concentration was observed when cuprophane (bio-incompatible membrane) was used during dialysis (F, in minutes), while such an increase was not observed when bio-compatible membranes were used.

A possible explanation for this phenomenon is that, in the case of artificial kidney treatment with bio-incompatible membranes, in the blood that leaves the artificial kidney complement is activated as a result of which granulocytes aggregate, adhere and degranulate in the first capillary bed they encounter, i.e. the microcirculation of the lung, thereby releasing generated free oxygen radicals. This may result in a local inflammation, edema formation, increase of the alveolar arterial oxygen tension difference and hypoxemia.

2. $\beta2$microglobulin

Extracorporeal circulation with bio-compatible membranes

The present innovators have discovered that while using bio-incompatible membranes (cuprophane) for extracorporeal circulation, especially hemodialysis, the concentration of $\beta_2$ microglobulin in the serum displays the same behaviour as described above for placental alkaline phosphatase.

Fig.3 shows the course of the $\beta_2$ serum microglobulin concentration expressed as a percentage of the pre-dialysis value with the duration of the dialysis (H, in hours) as function of the type of hemodialysis membrane used (o_____o cuprophane, x_____x modified cellulose, ●_____● polyacrylonitrile). After four hours of hemodialysis using a bio-incompatible membrane (cuprophane), the serum $\beta_2$ microglobulin concentration increased by 34.5 ± 12% (p < 0.01). Using modified cellulose as a bio-compatible membrane, after four hours of dialysis the serum $\beta_2$ microglobulin concentration increased by only 11.5 ± 6.5%, but no complement activation delevoped. Using the bio-compatible membrane made out of polyacrylonitrile a decrease of the serum $\beta_2$ microglobulin concentration of 11.5 ± 6.5% (p < 0.05) occurred.

A comparison between fig. 2 and 3 shows that the serum $\beta_2$ microglobulin concentration displays the same be haviour as placental alkaline phosphatase during hemodialysis.

Noticeable here is that the placental alkaline phosphatase in the lung is localized substantially at the epithelium of the respiratory bronchiolus and at the plasma membrane of type I epithelial cells (membranous pneumocytes) (E.J. Nouwen et al, Cancer Research 46, 866-876, 1986), while $\beta_2$ microglobulin is present in the lung at the plasma membrane of the endothelial cells (M. Governa et al, European Journal of Immunology 6, 830-832, 1976).

Applicable in each of the clinical situations described is that cytotoxic oxides are generated by activated inflammatory cells such as macrophages and neutrophils and by oxidation-reduction cycles for paraquat.

### 3. Immunological determination of the antigens

The antigens discussed above are determined using immunological techniques. Use can thereby be made of liquid or solid phases. EAIA, ELISA and RIA have the preference as immunological techniques, although immune diffusion or - electrophoresis allow the determination of antigens with specific antibodies.

In principle, use can be made of kits available on the market for determination of antigens. For human placental alkaline phosphatase for example the kit from Innogenetics, Antwerp, Belgium, and for $\beta_2$ microglobulin a kit from Pharmacia, Uppsala, Sweden.

As far as human placental alkaline phosphatase is concerned it is preferable to determine this antigen with a specific monoclonal antibody, because in the biological material, especially serum, other isoenzymes with a comparatively higher enzyme activity are also present.

### Human placental alkaline phosphatase-assay

Human placental alkaline phosphatase was isolated from five placentas. The placenta tissue was homogenized with 50 mM tris-HCl 1 mM MgSO$_4$, pH 6.8 (1/5, w/v) and extracted with n-butanol (2/5, v/v for the total homogenized material). The extract was centrifuged for 20 minutes at 1000 x g and the watery layer was collected. Subsequently acetone was added at -20°C in such a quantity that an acetone solution of about 30% was obtained. This solution was stirred for 20 minutes and then centrifuged for 20 minutes at 1000 x g. The supernatant was collected after which acetone was added in such a quantity that an acetone solution of about 50% was obtained. This solution was stirred for 20 minutes and again centrifuged. The precipitate was dissolved in 0.02 M tris-HCl, 0.5 M NaCL, pH 7.4. The raw enzyme preparation (100 ml, 6000 U) was subsequently placed on a column of Concanavaline A-Sepharose 4B (20 ml, Pharmacia, Uppsala, Sweden) after which the column was washed with 100 ml buffer. The glycoproteins were washed out of the column with 50 mM $\alpha$-D-methyl-glycoside (Sigma, Richmond, USA) in the above mentioned tris buffer. The fractions displaying alkaline phopshatase activity were pooled and transferred by dialysis with a XM-50 membrane (Amicon, Danvers, USA) into a 20 mM tris-HCl pH 7.7, 1 mM MgSO$_4$ buffer. These fractions were placed on a Mono Q HR (5/5) FPLC column (Pharmacia, Uppsala, Sweden) which had been equilibrated with the same buffer. Subsequently, the alkaline phosphatase activity was eluated from the column with a gradient of 20 mM tris-HCl pH 7.7 to 20 mM tris-HCl pH 7.7, 0.35 M NaCl. The fractions containing enzyme were dialysed with an XM-50 membrane against a physiological saline solution after which they were placed in fractions of 100 $\mu$l on a TSK G-3000 SW column (Toyo Soda, Japan) which was equilibrated with 0.9% NaCl. The fractions containing alkaline phosphatase were pooled, in concentrated form if necessary, and used for the immunization of Balb/c mice. The final yield amounted to 10% and had an enzyme activity of 220 U/mg. Rechromatography on TSK G-3000 SW as well as SDS-PAGE demonstrated that the isolated placental alkaline phosphatase was at least 95% pure.

The immunization was performed on Balb/c mice of 6 to 8 weeks of age. The first injection consisted of an emul sion of 1 part (100 $\mu$l) complete Freund adjuvans (Gibco, Paisley, UK) and 1 part placental alkaline phosphatase in physiological salt (1 mg/ml). Half of this emulsion was injected intradermally, the other half intraperitoneally. Booster injections with the same amount of antigen in incomplete Freund adjuvans (Gibco, Paisley, UK) were given intraperitoneally at intervals of two weeks. After the titre exceeded the value 1/10,000, 50 $\mu$g placental alkaline phosphatase in physiological salt was injected in the tail vena together with a same amount intraperitoneally three to four days before the fusion-experiment. A similar injection procedure was repeated the day before the fusion-experiment.

For the experiment, use was made of the myeloma cell line Sp2/0 (obtainable from Flow Cat no. 05-554 Sp2/0-Ag-14 clone of Sp2/HL-Ag myeloma). The cells which were in the logarithmical growth phase were removed from the surface of their culture glasses and resuspended in 10 ml Dulbecco's minimal essential medium (Gibco). The immunized Balb/c mice were killed, the spleen was removed and converted into a cell suspension in Dulbecco's minimal essential medium. The cell suspension was centrifuged for 10 minutes at 300 x g and resuspended in 10 ml Dulbecco's minimal essential medium.

The cells were mixed in a proportion of 1 myeloma cell to 2 spleen cells and centrifuged for 10 minutes at 300 x g. One ml polyethyleneglycol solution (PEG 4000, ± 35% solution in Dulbecco's minimal essential medium) was added to the cell pellet and lightly shaken, so that all the cells came into contact with polyethyleneglycol. The cells are then centrifuged again for three to five minutes at 300 x g. Eight minutes after the addition of polyethyleneglycol the pellet was diluted and resuspended in 10 ml Dulbecco's minimal essential medium, after which 15 ml Dulbecco's minimal essential medium was added. Following this the cells were centrifuged for 10 minutes at 300 x g and resuspended in Dulbecco's minimal essential medium +20% foetal calf serum. Finally the cells were diluted to a concentration of $5 \times 10^5$ cells per ml in a medium containing 50% HAT (2X) Dulbecco's minimal essential medium -20% foetal calf serum and 50% conditioned Dulbecco's minimal essential medium -20% foetal calf serum. The HAT medium was prepared by dissolving 17.6 mg aminopterin in 10 ml 0.1 M NaOH, neutralising it with HCl, and supplementing it to 100 ml with distilled water (solution A). Separately 0.136 g hypoxantin was dissolved in water and 0.0387 g thymidine was added and dissolved (2 ml was added to 100 ml medium) (HT solution). For the preparation of the HAT medium 10 ml solution A was added to the HT solution, water was added to 200 ml and sterilization was subsequently carried out.

Subsequently the cells were plated, whereby each well was filled with 1 ml cell suspension. After 14 days the control myeloma cells died and aminopterin could be removed. Hybrids for the specific monoclonal antibodies developed and were repeatedly sub-cloned.

A monoclonal antibody which was used for the immunological determination, displayed a cross reactivity for aspecific alkaline phosphatase and for intestinal alkaline phosphatase of less than 0.01%. This monoclonal antibody belongs to the IgGI immunoglobulin sub-class.

If an interference as a result of cross reactivity is to be expected, this interference can be avoided by heating the samples for 10 minutes at 65°C, because only placental alkaline phosphatase can endure such a heat treatment.

For the quantitative determination of placental alkaline phosphatase an enzyme-antigen immuno-assay (EAIA) was set up in which polyclonal rabbit anti-serum against mouse immunoglobulins was adsorbed on microplates, to which the monoclonal antibody was then added. The sample containing the placental alkaline phosphatase is subsequently allowed to react for some time with the solid phase bonded monoclonal antibody. An extract obtained by butanol extraction from biological tissue can also be used as sample (Pollet et al, Clinic Chemistry 31, 41, 1985).

## Claims

1. Method for diagnosis and/or prospective following of injury at the point of the pulmonary microcirculation, comprising the immunological determination in biological material originating from the patient of antigen or antigens secreted by the alveolar lumina at the point of said pulmonary microcirculation under influence of this injury.

2. Method as claimed in claim 1 in which the antigen is placental alkaline phosphatase.

3. Method as claimed in claim 1 or 2 in which the antigen is $\beta_2$ microglobulin.

4. Method as claimed in claims 1-3, characterized in that the antigen or the antigens is/are determined with a monoclonal antibody.

5. Method as claimed in any of the foregoing claims, characterized in that the biological material comprises blood, serum, plasma, pulmonary fluid, a lung biopsy or a tissue extract.

6. Method as claimed in any of the foregoing claims, characterized in that the immunological determination is performed with a RIA, ELISA, EAIA, IRMA and the like.

7. Method as claimed in claims 1-6 for diagnosis or prospective following of an acute respiratory distress syndrome (ARDS) in adults and children.

8. Method as claimed in claims 1-6 for diagnosis or prospective following of injury at the point of the pulmonary microcirculation by artifical ventilation.

9. Method as claimed in claims 1-6 for diagnosis or prospective following of an intoxication by a compound causing pulmonary oxygen intoxication.

10. Method as claimed in claim 9, characterized in that the compound is paraquat.

11. Method as claimed in claims 1-9, for diagnosis and/or prospective following of injury at the point of the pulmonary microcirculation by extracorporeal circulation with bio-incompatible membranes.

12. The use of a specific antibody for an antigen which is secreted by the alveolar lumina in a biological material under the influence of injury at the point of the pulmonary microcirculation, for the manufacture of a diagnostic kit for diagnosis and/or prospective following of this injury at the point of said pulmonary microcirculation.

13. The use as claimed in claim 12, in which the antigen is placental alkaline phosphatase.

14. The use as claimed in claims 12-13, in which the antigen is $\beta_2$ microglobulin.

15. The use as claimed in claims 12-14, characterized in that the antigen is determined with a monoclonal antibody.

16. The use as claimed in claims 12-15, characterized in that the biological material comprises blood, serum, plasma, pulmonary fluid, a lung biopsy or a tissue extract.

17. The use as claimed in claims 12-16, characterized in that the immunological determination is performed with a RIA, ELISA, EAIA, IRMA and the like.

18. The use as claimed in claims 12-17, for diagnosis or prospective following of the acute respiratory distress syndrome (ARDS) in adults and children.

19. The use as claimed in claims 12-17, for diagnosis or prospective following of injury at the point of the pulmonary microcirculation by artificial ventillation.

20. The use as claimed in claims 12-17, for diagnosis and/or prospective following of an intoxication by a compound causing pulmonary intoxication.

21. The use as claimed in claim 20, characterized in that the compound is paraquat.

22. The use as claimed in claims 12-17, for diagnosis and/or prospective following of injury at the point of the pulmonary microcirculation by extracorporeal circulation with bio-incompatible membranes.

FIG.1

FIG.2

FIG.3